# EUROPÄISCHE PATENTSCHRIFT

(11) **EP 0 774 927 B1**
(45) Veröffentlichungstag und Bekanntmachung des Hinweises auf die Patenterteilung: **19.08.1998**
(21) Anmeldenummer: 94912482.0
(22) Anmeldetag: 23.04.1994
(51) Int. Cl.: A61B 19/00, A61N 7/00

(54) **VORRICHTUNG ZUR BEHANDLUNG VON KRANKHAFTEN ZELLEN IM LEBENDEN KÖRPER**
DEVICE FOR TREATING MORBID CELLS IN THE LIVING BODY
DISPOSITIF DE TRAITEMENT DE CELLULES MORBIDES DANS LE CORPS VIVANT

(30) Priorität: 24.04.1993 DE 4313483; 13.04.1994 DE 4412652
(43) Veröffentlichungstag der Anmeldung: 28.05.1997
(73) Patentinhaber: THEUER, Erich, D-73773 Aichwald (DE)
(72) Erfinder: THEUER, Erich, D-73773 Aichwald (DE)
(74) Vertreter: Patentanwälte Eisele, Otten & Roth
(86) Internationale Anmeldenummer: DE9400451
(87) Internationale Veröffentlichungsnummer: WO9424953

(56) Entgegenhaltungen:
- EP-A- 0 332 871
- WO-A-89/02724
- WO-A-92/07622
- DE-A- 3 713 816
- US-A- 4 315 514

## Beschreibung

Die Erfindung betrifft eine Vorrichtung zur Behandlung von krankhaften Zellen und malignen Geweben im lebenden Körper.

Nicht nur im Bereich der medizinischen Diagnose, sondern auch für medizinische Therapien werden in vielerlei Hinsicht Bestrahlungen mit Ultraschallwellen eingesetzt. Hierzu wird beispielsweise auf die EP 0 324 948 A2 oder die DE 39 00 433 C2 verwiesen.

Insbesondere ist die therapeutische Anwendung von Ultraschall im Bereich der Chirurgie und Orthopädie, der Inneren Medizin, der Neurologie, bei Erkrankungen des rheumatischen Formenkreises sowie bei verschiedenen weiteren Krankheitsbildern bekannt geworden.

Die Wirkungsweise des Ultraschalls beruht auf dem Zusammentreffen mehrerer Effekte. Im Vordergrund steht hierbei die mechanische Wirkung, d. h. durch die Zug- und Druckwirkung der Schalleinstrahlung, die, wie allgemein bekannt, in Form longitudinaler Druckwellen vorliegt, werden Zellelemente in Bewegung gebracht. Die mechanische Wirkung von Ultraschalleinstrahlung wird daher zutreffend auch mit dem Begriff "Mikromassage" bezeichnet. Diese innere Gewebemassage bewirkt bei geringer Dosierung eine Förderung des Zellstoffwechsels, der Durchblutung und der Sauerstoffversorgung.

Eine weitere Besonderheit stellt die Wärmewirkung beim Ultraschall dar. Es kommt hierbei zu einer Umwandlung von Bewegungsenergie in Wärme. Die Intensität der eingestrahlten Energie nimmt durch Absorption bei zunehmender Gewebetiefe stark ab. Die Wärmewirkung ist von der Intensität abhängig und hat bei niedriger Dosierung keine große Bedeutung während bei einer Einstrahlung mit 2 bis 3 Watt pro cm² bereits eine wesentlich stärkere Wärme entsteht.

Zudem gelten gewisse chemische Reaktionsabläufe unter der Einwirkung von Ultraschalleinstrahlungen heute als bewiesen. Dazu gehört beispielsweise die Diffusionsfähigkeit verschiedener Substanzen durch Membranen oder durch die intakte Haut. Änderungen vom Gel- in den Solzustand sind durch Ultraschall zu erreichen. Die Leitfähigkeit elektrolytischer Lösungen nimmt zu, Oxydationsprozesse werden ausgelöst, chemische Reaktionen laufen wie unter katalytischen Bedingungen ab. Hinzu kommt eine gewisse Verschiebung des PH-Wertes im Gewebe in die alkalische Richtung.

Neben mechanischen, thermischen und chemischen Wirkungen muß die Reaktionsweise isolierter Organe sowie des gesamten Organismus herausgestellt werden. Diese biologische Wirkungsweise kommt auf dem Wege neurohumoralen Zusammenspiels zustande. Die biologische Wirkungsweise ist für die Praxis von großer Bedeutung. Jede Änderung der physikalischen Parameter, z. B. Frequenz, Intensität, Beschallungsdauer, zieht Veränderungen der mechanischen, thermischen und chemischen Wirkungsweise nach sich und bedingt eine veränderte biologische Reaktion. Die biologische Reaktion auf Ultraschalleinstrahlung unterliegt äußerst komplexen Wirkungsmechanismen.

So beschreibt die US-A-4315514 eine Vorrichtung zur Behandlung von krankhaften Zellen und Geweben mittels Ultraschallwellen mit einer Steuer- und Regeleinheit, welche mit dem eine Schallquelle umfassenden Behandlungsgerät zur selektiven Zerstörung krankhafter Zellen verbunden ist, wobei dazu die betreffende Zellenart zerstörende spezifische Resonanzfrequenzen in einer entsprechenden letalen Bestrahlungsdosis verwendet werden.

Allen Ultraschallgeräten für die bislang bekannten Therapieanwendungen sind folgende Eigenschaften gemeinsam:

Die abstrahlbare Leistung ist sehr niedrig, sie bewegt sich in der Größenordnung von einigen Watt pro cm², vorzugsweise unterhalb von 2 Watt pro cm².
Die abgestrahlten Ultraschallfrequenzen liegen vergleichsweise niedrig, d. h. in einem Frequenzbereich unterhalb von ein MHz.
Impulsschallverfahren sind möglich, wobei die Impulsdauer sich in der Größenordnung von 1 bis 10 ms bewegt und die Pausendauer unterhalb von 50 ms liegt.
Durch die lokal eingeschränkten Anwendungsmöglichkeiten werden Ultraschallköpfe mit kleinen effektiv abstrahlenden Flächen in der Größenordnung von einigen cm², vorzugsweise unterhalb von 5 cm², verwendet.

Gegenüber bekannten Anwendungen mit derartigen Geräten, bei denen die oben angeführten Wirkungsweisen mehr oder weniger zur Stimulierung des bestrahlten Gewebes eingesetzt werden, soll durch die vorliegende Erfindung eine Vorrichtung bzw. eine Anordnung geschaffen werden, mittels welcher Ultraschallenergie zur selektiven Zerstörung von Zellen mit einem bestimmten krankhaften Erscheinungsbild, beispielsweise von Tumorzellen, eingesetzt wird. Erfindungsgemäß muß die Schallwelle der hierzu konzipierten Vorrichtung zur selektiven Zerstörung derartiger Zellen so ausgelegt sein, daß es Schall- und/oder Ultraschallwellen mit einem für die jeweilige Zellenart typischen, diese Zellen zerstörenden Resonanzfrequenzspektrum erzeugen kann.

Der vorliegenden Erfindung liegt die Erkenntnis zugrunde, daß für ein mögliches Resonanzverhalten von Körperzellen die Zellengröße nicht allein von maßgeblicher Bedeutung ist. Das Schwingungsverhalten wird vielmehr von dem osmotischen Druck, der Kerngröße der Beschaffenheit des Zytoskeletts, der Gewebetemperatur, der Vordehnung der Mikrofilamente und dem interzellulären Skelett geprägt.

So weisen z. B. Krebszellen eine prägnante Vergrößerung des Zellkerns auf. Dabei bleibt das Verhältnis Kerngröße zu Zellgröße unverändert. Der Zellkern ist von Zellflüssigkeit umgeben, die in einer Zellmembran eingeschlossen ist. Die Zellflüssigkeit weist kolloidale Eigenschaften auf. In ihr befinden sich sogenannte Zellfilamente, die den Zellkern positionieren und Kräfte zwischen der Zellmembran und dem Zellkern übertragen. Hierdurch ist es möglich, durch spezielle Bestrahlungsfrequenzspektren die Zellkerne derart in Schwingung zu versetzen, daß die von den Zellfilamenten auf die Zellmembran übertragenen Kräfte die Membran derart schädigen, daß die Zelle platzt und somit zerstört wird. Man spricht hier von einem sogenannten "stress stiffing" der Zellmembran.

Die typischen Strahlungsspektren können beispielsweise durch Computersimulationsanalysen der Schwingungseigenschaften von gesunden und krankhaft veränderten Zellen berechnet werden. Hierbei werden das viskoelastische Verhalten der Zellwand, die Fließ- und Dämpfungseigenschaften der Zellflüssigkeit und die Kompressibilität des Zellkerns und des Zytoplasmas berücksichtigt. Zusätzlich werden sowohl die Eigenschaften des Zytoskeletts und der osmotische Druck als auch die vorliegenden thermischen Potentialfelder einbezogen.

Von großem Vorteil ist es bei der Verwendung einer erfindungsgemäßen Vorrichtung, daß es Bestrahlungsspektren gibt, bei denen gesunde, normale Zellen Belastungen unterliegen, die sie entweder gar nicht oder nur in erträglichem Maße schädigen.

Da die einzustrahlenden Frequenzspektren sehr typisch für die jeweils zu zerstörenden Zellenarten sind, d. h. die Frequenzspektren können je nach Anwendung bei verschiedenen Tumorarten sehr differieren, ist es vorteilhaft, daß die Bestrahlungsvorrichtung mit einer oder mehreren Frequenzlinien und/oder einem oder mehreren Frequenzbändern innerhalb eines breiten Frequenzbereichs verstellbar ist. Ein derartig breiter Frequenzbereich liegt typischerweise zwischen 1 kHz und 10 MHz.

Um alle gewünschten Frequenzspektren einstellen zu können, ist es empfehlenswert, daß verschiedene Frequenzbänder und/oder -linien überlagerbar sind. Durch die Möglichkeit verschiedene Frequenzlinien bzw. Frequenzbänder zu überlagern und gleichzeitig diese Frequenzlinien bzw. Frequenzbänder abzustimmen, kann das Frequenzspektrum auf alle möglichen Arten von krankhaften Zelltypen eingestellt werden. Dies ist erforderlich, da wie oben bereits erläutert, die Wirkungsweise der eingestrahlten Schall- und/oder Ultraschallstrahlung auf einem komplexen Reaktionsmechanismus beruht und somit jeder Zellentyp eine Einstrahlung mit einem auf ihn abgestimmten Frequenzspektrum erfordert. Ein erfindungsgemäßes Bestrahlungsgerät mit einer breiten Variabilität in seinen Einstellwerten kann ein entsprechend breites Anwendungsgebiet bei einer Vielzahl verschiedener Zelltypen finden.

Vorteilhaft ist es, wenn eine oder mehrere Frequenzlinien und/oder Frequenzbänder in ihrer Amplitude und Breite variabel sind. Die vom Schallkopf abgestrahlte Leistung sollte hier möglichst bis zu 300 Watt pro cm² betragen. Dies ist wichtig, um genügend Energie zur Zerstörung der selektierten Zellen einstrahlen zu können.

Vorzugsweise wird die Bestrahlung in Form von Impulsfolgen realisiert. Ein hierzu verwendbares erfindungsgemäßes Stoßwellengerät sollte wiederum in seiner Impulsdauer und/oder in seiner Pausendauer verstellbar sein. Die Bestrahlung in Form von Stoßwellen hat den Vorteil, daß die mit der Einstrahlung von Schall- und/oder Ultraschallwellen verbundene Energieabsorption innerhalb des Gewebes zu keiner übermäßigen Temperaturerhöhung führt. Impulsdauer und Pausendauer einer derartigen Impulsfolge sollten dementsprechend nicht nur im Hinblick auf einen optimalen Therapieerfolg bei der Zerstörung der krankhaften Zellen sondern auch im Hinblick auf eine möglichst gleichförmige, für gesundes Zellgewebe verträgliche Umgebungstemperatur ausgerichtet sein.

Typischerweise sind die Impuls- und/oder die Pausendauern der Impulsfolgen zwischen 0,1 s und 120 s variierbar.

Des weiteren ist es von großem Vorteil, wenn ein wasserdichter Schallkopf als Schallquelle beispielsweise für eine Unterwasseranwendung eingesetzt wird. Vor allem dann, wenn eine Schallquelle mit großer Abstrahlfläche verwendet wird, so daß ein menschlicher Körper oder zumindest ein ganzes Körperteil wie der Kopf, der Bauch etc. bestrahlbar sind, kann die Ankopplung der Schall- und/oder Ultraschallstrahlung an den entsprechenden Körper bzw. das entsprechende Körperteil unter Wasser besser bewerkstelligt werden.

Um ganze Körperteile zu bestrahlen, wäre auch das Abrastern mit einem Schallkopf mit kleiner Abstrahlfläche denkbar. Vorteilhafterweise wird hierzu ein steuerbarer Führungsmechanismus für einen derartigen Schallkopf verwendet, um den zu bestrahlenden Körper bzw. das entsprechende Körperteil abzurastern.

Für eine lokale Anwendung innerhalb des Körpers kann ein Schallkopf auch in seiner äußeren Form so angepaßt werden, daß er in Körperöffnungen und/oder Körperkanäle wie Nase, Luftröhre, Blutgefäße etc. eingeführt werden kann. Dies ist insbesondere bei Behandlung von weit innenliegenden Tumoren, wie beispielsweise Gehirntumoren etc., von Vorteil, da hier über einen in den Körper einführbaren Schallkopf die Schallquelle in direkte Nähe des zu bestrahlenden krankhaften Gewebes gebracht wird und somit die volle Abstrahlleistung auf das zu zerstörende Gewebe trifft.

Die erfindungsgemäße Vorrichtung wird insbesondere durch ein Aggregat für die Voll- und Teilunterkühlung ergänzt, um eine möglichst geringe Viskosität der Zelle vorzubereiten.

Weiterhin kann die Vorrichtung ein Gerät zur Echoauswertung umfassen, um eine Frequenzanalyse des beschallten Gewebes vornehmen zu können. Mittels eines weiterhin zugeordneten Simulationsrechners wird die erforderliche Beschallungsintensität bestimmt.

Weitere Besonderheiten der Erfindung werden nachfolgend anhand der Figuren der beigefügten Zeichnungen als beispielhafte Ausführung der Erfindung erläutert.

Es zeigen:
- Fig. 1: eine Anordnung der Gerätekomponenten zur Darstellung der erfindungsgemäßen Vorrichtung,
- Fig. 2: das visko-elastische Deformationsverhalten einer Zelle
- Fig. 3: ein Beispiel eines aus mehreren Frequenzbändern zusammengesetztes Frequenzspektrum und
- Fig. 4: zwei mögliche Ausführungen einer erfindungsgemäßen Impulsfolge.

### Beschreibung der Erfindung:

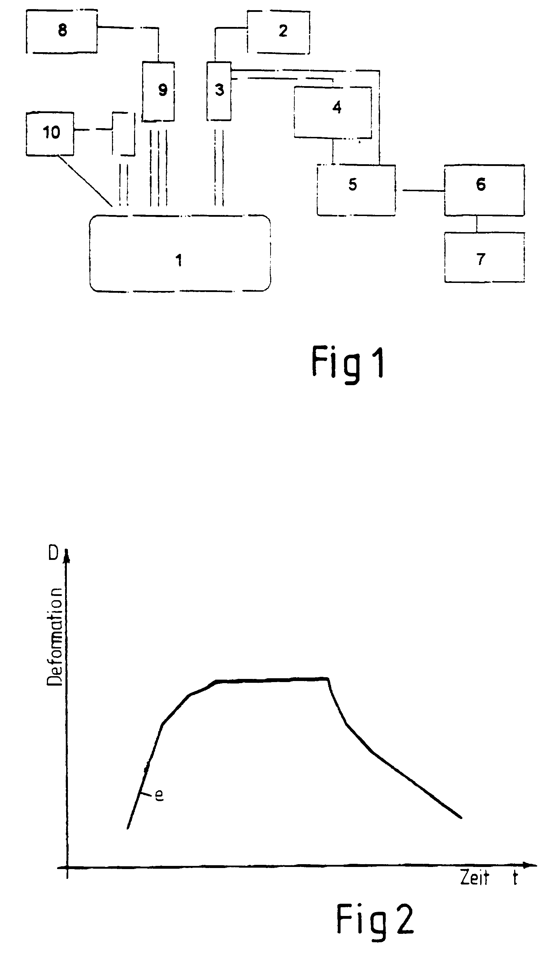

Wie sich aus der Darstellung nach Fig. 1 ergibt, ist die erfindungsgemäße Vorrichtung aus nachfolgend erwähnten Aggregaten sinnvoll und je nach Anwendungsfall aufgebaut. Neben dem RF-Generator 2 (Resonanzfrequenz-Generator) zur Erzeugung des Ultraschalls und den Ultraschall-Köpfen 3 für die Beschallung des Gewebes umfaßt die erfindungsgemäße Vorrichtung einen Behandlungstisch 1 mit einer Einrichtung zur Voll-, und/oder Teilunterkühlung des zu behandelnden Gewebes. Weiterhin ist ein Gerät 4 für die Echoauswertung der eingestrahlten Ultraschall-Schwingungen sowie ein Laser-Interferometer 10 für die Messung der Amplituden einer fixierten Tumorzelle oder eines Gewebebereiches vorgesehen. Schließlich kann ein Simulationscomputer 7 mit A/D Konverter für die Bestimmung der letalen Beschallungsintensitäten verwendet werden.

Besonders vorteilhaft für Schwingungen mit ausreichender Ausschlagamplitude der malignen Zellkerne ist eine möglichst steife Beschaffenheit des Zytoskelettes. Mit der erfindungsgemäßen Vorrichtung wird das sich bei Belastung versteifende, nichtlineare Spannungs-Dehnungsverhalten der Aktin- und Vimentinfilamente des Zytoskelettes ausgenutzt und das Beschallungsaggregat derart gestaltet, daß vor der eigentlichen Ultraschall-Behandlung eine niederfrequente Ultraschall-Vorbestrahlung erfolgt, die eine Vordehnung und damit eine Verfestigung des malignen Zytoskelettes bewirkt. Hierfür ist der RF-Generator 8 mit Ultraschall-Kopf 9 für eine niederfrequente Vorbestrahlung vorgesehen.

Vorteilhaft für ein Schwingen mit ausreichender Ausschlagamplitude der malignen Zellkerne ist weiterhin eine möglichst geringe Viskosität der Zelle. Dies wird dadurch geschaffen, daß die erfindungsgemäße Vorrichtung einen Behandlungstisch 1 mit einer Möglichkeit zur Unterkühlung zunächst aller zu beschallenden Gewebeteile umfaßt, wodurch ein zähflüssiges Zytoplasma erzeugt wird. Die Unterkühlung erfolgt derart, daß ein Resonanzschwingen dann nicht mehr möglich ist, da die angeregten Schwingungen überdämpft sind.

Tumorzellen bzw. maligne krankhafte Zellen weisen einen überaktiven Stoffwechsel auf, der zu einem gegenüber benachbarten gesunden Zellen vorzeitigen Temperaturanstieg mit abnehmender Viskosität der malignen Zelle führt. Dies wird mit der erfindungsgemäßen Vorrichtung ausgenutzt, indem allein die Krebszellen auf die Ultraschall-Beschallung reagieren, während gesunde benachbarte Zellen aufgrund der noch fortbestehenden Unterkühlung durch eine Überdämpfung nicht reagieren. Die Anregung des Stoffwechsels des malignen Gewebes kann z. B. durch eine metabolismusanregende Stimulation oder durch eine sonstige Erwärmung der Tumorzellen erfolgen. Hierdurch werden maligne Zellen zu Resonanzamplituden angeregt und letal geschädigt.

Die erfindungsgemäße Vorrichtung kann auch ein Gerät 4 für die Echoauswertung des beschallten Gewebes umfassen. Mit Hilfe des Gerätes 4 für die Echoauswertung wird nach jedem Impuls eine Frequenzanalyse des beschallten Gewebes vorgenommen. Das Gerät ist so konzipiert, daß nach der Analyse der Echosignale und Sichtbarmachung der Resonanzfrequenzbänder eine sofortige Anpassung der Beschallung auf die jeweilige Resonanzfrequenz der malignen Zellen vorgenommen wird.

Die erforderliche Beschallungsintensität zur letalen Schädigung der Krebszellen wird über ein FEM-Computersimulationsmodell auf einem integrierten, zum Behandlungsgerät gehörenden Simulationsrechner bestimmt. Das FEM-Simulationsmodell beinhaltet die malignen Zellen des beschallten Gewebes mit den malignen Mikrofilamenten, dem malignen Zytoplasma, der malignen Zellmembran und dem malignen interzellulären Skelett sowie die gesunden Zellen des beschallten Bereiches mit ihren Organen wie Zellkern, Zytoskelett, Zytoplasma, Zellmembran und dem interzellulären Skelett. Auf dem Rechner wird simultan eine plastischdynamische Belastungsanalyse durchgeführt mit deren Hilfe die maximalen Deformationen und Dehnungen in der malignen Zellmembran bestimmt werden und die erforderlichen Beschallungsleistungen für eine letale Schädigung der malignen Zellmembran festgelegt werden.

Das Ultraschall-Bestrahlungsgerät 2, 3 kann auch ferner durch ein Laser-Interferometer 10 ergänzt werden, mit dessen Hilfe eine Fixierung und Messung der Resonanzamplituden der Tumorzellen oder ein Bereich des beschallten Gewebes erfolgt. Die Ausschlagamplituden werden über den reflektierten Laserstrahl gemessen und über ein bildgebendes Verfahren sichtbar gemacht. Daraufhin kann die Beschallungsfrequenz auf die jeweilige Resonanzfrequenz der erkrankten Zellen eingestellt werden.

Für die selektive, schwingungsinduzierte Zerstörung maligner Zellen wird demzufolge eine Vorrichtung bzw. Anlage oder Anordnung vorgeschlagen, die sich aus verschiedenen, erfindungsspezifisch aufeinander angepaßten Einzelaggregaten bzw. Geräten zusammensetzt, wie dies insbesondere in der Fig. 1 wiedergegeben ist. Neben dem RF-Generator 2 zur Erzeugung der Resonanzfrequenz (RF) und den Ultraschall-Köpfen 3 für die Hauptbehandlung der malignen Zellen ist ein weiterer spezieller RF-Generator 8 mit Ultraschall-Köpfen 9 für die niederfrequente Vorbestrahlung der malignen Zellen vorgesehen. Weiterhin umfaßt die Vorrichtung - wie erwähnt - das Aggregat 1 mit Behandlungstisch für die Unterkühlung des befallenen Körperteils, das Gerät 4 für die Echoauswertung der reflektierten Schwingungen, das Laser-Interferometer 10 für die Messung der Resonanzamplituden einer fixierten Zelle des behandelten Gewebes und einen Simulationscomputer 7 für die simultane Bestimmung der letalen Beschallungsintensitäten.

Weiterhin ist in Fig. 1 ein sogenanntes lineares Tor 5 zur Signalausblendung dargestellt, welches mit dem Ultraschallsignalaufnehmer 4 zusammenwirkt. Weiterhin sind ein Spektralanalysator 6 mit A/D Konverter + Simulationscomputer 7 dem Gerät 5 nachgeschaltet. Die jeweiligen RF-Generatoren 2, 3 sind den Ultraschall-Köpfen 3, 9 zugeordnet.

Die erfindungsgemäße Vorrichtung nach Fig. 1 mit den einzelnen Aggregaten ermöglicht demzufolge eine selektive Zerstörung maligner Tumorzellen, wobei gesunde, normale Zellen die Beschallung ohne Schaden zu nehmen überleben. Dabei ist eine großflächige, nicht fokussierte selektive Zerstörung maligner Tumorzellen möglich. Weiterhin kann mit der Vorrichtung eine sanfte Beschallung auch bei extrem niedrigen Bestrahlungsleistungen vorgenommen werden, wodurch Schäden durch ungewollte Kavitation vermieden werden.

Die Vorrichtung nach Fig. 1 mit den darin enthaltenen Aggregaten ermöglicht insbesondere eine Vorbeschallung mit Vordehnung und lokaler Erwärmung der malignen Zellen. Hierdurch kommt es zu einer selektiven Reduzierung der malignen Zellviskosität bei gleichzeitiger Skelettverfestigung und zu einer drastischen Erhöhung der malignen Resonanzamplituden.

Es ist auch möglich, eine selektive Erhöhung des osmotischen Druckes maligner Zellen zu erzielen. Weiterhin ermöglicht die Anordnung nach Fig. 1 eine genaue Einstellung der letalen Beschallungsleistung mit Hilfe von Computersimulationsmodellen. Schließlich ist auch eine exakte letale Beschallungsdauer erzielbar.

In der Fig. 2 ist das visko-elastische Deformationsverhalten einer Zelle gezeigt.

Maligne als auch gesunde Zellen weisen bei der Belastung ein visko-elastisches Verhalten auf, welches in Fig. 2 dargestellt ist. Dabei weist das Deformations-Zeit-Diagramm in der Anfangsphase einer plötzlich aufgebrachten Belastung einen linear-elastischen Bereich "e" auf. Danach kommt es zu einer nicht linearen viskosen Deformation. Voraussetzung für ein nichtüberdämpftes Schwingen ist dabei eine Anregung, die Deformationen im elastischen Bereich des visko-elastischen Verformungsdiagrammes bewirkt.

Eine selektive Zerstörung maligner Zellen wird weiterhin durch eine selektive Erwärmung bewirkt. Dies kann entweder durch eine vorausgehende Unterkühlung mit anschließender metabolismusanregender Medikation sein, oder es kann eine Vorbehandlung mit Ultraschall durchgeführt werden. Dabei muß die Beschallung so abgestimmt sein, daß eine selektive Erwärmung der malignen Tumorzellen möglich ist. Anschließend erfolgt die Hauptbeschallung, die zu einem Resonanzschwingen der malignen Zellen und ihrer letalen Schädigung führt.

Bei der Behandlung mit Ultraschall treten dabei oft ungewollte Kavitationseffekte auf. Dabei kann es zu Schädigungen des gesunden Gewebes kommen. Eine kavitationsfreie Beschallungsleistung kann dann erreicht werden, wenn durch Vorbehandlung und exakte Ermittlung der Resonanzfrequenzen ausreichend niedrige Leistungen mit letalen Folgen möglich sind.

Die in Fig. 1 dargestellten Aggregate erlauben demzufolge eine Belastung des Zytoskelettes im elastischen Bereich des visko-elastischen Deformationsverhaltens. Hierdurch werden gemäß der Darstellung nach Fig. 2 die Überdämpfungserscheinungen vermieden.

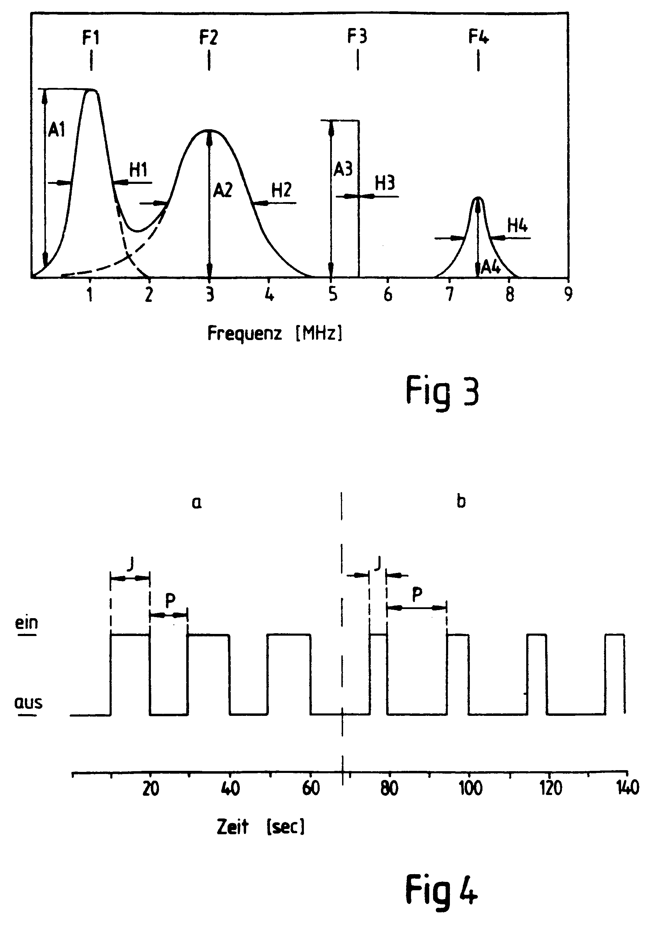

Die nachfolgend in den Figuren 3 und 4 dargestellten Frequenzverläufe werden bei der Ultraschallapplikation verwendet.

Im Diagramm gemäß Figur 3 sind vier verschiedene Frequenzbänder F1 bis F4 dargestellt. Sie weisen unterschiedliche Halbwärtsbreiten H1 bis H4 und verschiedene Amplituden A1 bis A4 auf. Die Form von erfindungsgemäß verwendungsfähiger Spektren ist keineswegs auf die gezeigte Darstellung mit separaten Frequenzbändern eingeengt. Die einzelnen Halbwärtsbreiten H1 bis H4 könnten beispielsweise so breit sein, daß verschiedene Frequenzbänder (F1 und F2) ineinander übergehen. Andererseits können einzelne Frequenzbänder F3 so schmalbandig, d. h. mit so kleiner Halbwärtsbreite H3 angewandt werden, daß sie im vorliegenden Maßstab nur als Frequenzlinien sichtbar sind. Die Anzahl der angewandten Frequenzbänder und/oder -linien kann ebenfalls schwanken. So kann beispielsweise in bestimmten Anwendungsfällen bereits eine einzige scharfe Frequenzlinie wirksam sein. In anderen Anwendungsfällen kann es unter Umständen notwendig werden, ein breit verteiltes Spektrum bis hin zu einer Gleichverteilung innerhalb eines großen Frequenzintervalls einzustrahlen. Selbstverständlich muß auch die Amplitude (A1 bis A4) einzelner Frequenzbänder und/oder -linien auf den jeweiligen Anwendungsfall abgestimmt werden.

Das einzustrahlende Frequenzspektrum hängt, wie oben erläutert, stark von der zu zerstörenden Zellenart ab. Je nachdem kann das anzuwendende Frequenzspektrum sogar von der Umgebung der krankhaften Zellen abhängen. Stehen beispielsweise mehrere mehr oder weniger wirksame Frequenzspektren zur Auswahl, so kann diese Auswahl mitunter durch die Wirkung des Frequenzspektrums auf das zu schonende Zellgewebe der Umgebung bestimmt werden.

Je nach Form des eingestrahlten Frequenzspektrums wird im bestrahlten Gewebe ein unterschiedlicher Energiebetrag absorbiert. Der absorbierte Energiebetrag äußert sich in Form einer Erwärmung des bestrahlten Gewebes. Um hierbei Schädigungen zu vermeiden, kann in Form von Impulsfolgen eingestrahlt werden.

Die z. B. möglichen Zeitabläufe zwei verschiedener Impulsfolgen sind in Figur 4 dargestellt. In den beiden durch eine gestrichelte Linie getrennten Teilbereichen a und b ist jeweils der Zeitverlauf dargestellt, mit dem die Einstrahlung ein- bzw. ausgeschaltet wird. Die Impulsbreite I und die Pausendauer p sind im Beispiel a gleich lang und betragen 10 s. Im Beispiel b beträgt die Impulsbreite I mit 5 s nur noch 1/3 der Pausendauer P (15 s). Die genannten Beispiele können in vielfältiger Weise variiert werden. Wie groß die Bereiche dieser Variationen ausgelegt werden müssen, wurde bereits weiter oben erläutert. Für bestimmte Anwendungsfälle können verschiedene Impulsfolgen auch aufeinanderfolgend kombiniert werden.

Die Flanken zwischem dem ein- und ausgeschalteten Zustand müssen keinesweg so steil sein, wie im dargestellten Beispiel. Denkbar wäre ebenfalls ein allmähliches Ein- und Ausschalten mit sanften Übergängen.

Bei derart steilen Flanken wie dargestellt, kann jedoch zusätzlich zur Temperaturkontrolle des bestrahlten Gewebes ein sogenannter Stoßwelleneffekt auftreten, der die zerstörende Wirkung des eingestrahlten Resonanzfrequenzspektrums unterstützt. Auch aus diesem Grund ist eine breite Variabilität der Impulsfolge für eine möglichst breite Anwendung notwendig.

Wie bereits erwähnt, können für eine Verbesserung der Wirkungsweise der erfindungsgemäßen Vorrichtung die zu zerstörenden Zellen auch medikamentös vorbereitet werden. Denkbar wären Medikamente, die die viskoelastischen Eigenschaften des Zytoplasmas verändern oder aber auch sogenannte Oxidantien, die die zu bestrahlenden Zellen mit Sauerstoff anreichern. Durch die Einwirkung der erfindungsgemäßen Bestrahlung wird anschließend die chemische Oxidation aktiviert, wodurch wiederum die entsprechenden Zellen zerstört werden. In diesem Zusammenhang wäre auch der Einsatz eines Sauerstoffgerätes in Kombination mit der Erfindung vorteilhaft.

Durch die Absorption innerhalb von gesundem Gewebe kann das eingestrahlte Frequenzspektrum mit fortschreitender Eindringtiefe verändert werden. Um dennoch auch tiefliegendes krankhaftes Zellgewebe mit der richtigen Frequenzauswahl zu bestrahlen, kann eine Art von "Frequenzstabilisatoren" in das Gewebe eingebracht werden. Hierunter sind kleine Partikel bestimmter Masse zu verstehen, die durch die eingestrahlten Druckwellen in Schwingung versetzt werden. Je nach Masse und Umgebung dieser Partikel weisen diese wiederum bestimmte bevorzugte Schwingungszustände (Resonanzlinien) auf. Derartige Partikel werden also auch wenn sie mit Frequenzen außerhalb ihrer Resonanzlinien beschallt werden einen gewissen Anteil von Schallwellem emittieren, die genau auf ihren Resonanzlinien liegen. Durch diese Vorgehensweise wird also das eingestrahlte Frequenzspektrum in tieferliegenden Bereichen quasi regeneriert.

Die Erfindung ist nicht auf das beschriebene Ausführungsbeispiel beschränkt. Sie umfaßt auch vielmehr alle fachmännischen Weiterbildungen und Verbesserungen im Rahmen der Offenbarung. Sofern mittels der erfindungsgemäßen Vorrichtung ein Arbeitsverfahren abläuft, so ist dies ebenfalls Gegenstand der vorliegenden Erfindung.

## Patentansprüche

1. Vorrichtung zur Behandlung von krankhaften Zellen und malignen Geweben im lebenden Körper mittels Ultraschallwellen mit einer Steuer- und/oder Regeleinheit und einem mit der Steuer- und/oder Regeleinheit verbundenen Beschallungsgerät (2, 3) mit wenigstens einer Schallquelle (3) zur selektiven Zerstörung von Zellen mit krankhaftem Erscheinungsbild, beispielsweise Tumorzellen, für die Erzeugung von Ultraschallwellen dadurch gekennzeichnet, daß die Vorrichtung für die Erzeugung einer Schall- und/oder Ultraschallstrahlung mit einem für die jeweiligen Zellenarten typischen, diese Zellen zerstörenden Resonanzfrequenzspektrum ausgelegt ist unter Verwendung einer selektiven, für maligne Zellen letalen Bestrahlungsleistung, wobei Mittel für die Überlagerung verschiedener Frequenzlinien (F1 bis F4) und/oder Frequenzbänder vorgesehen sind.

2. Vorrichtung nach Anspruch 1, dadurch gekennzeichnet, daß eine oder mehrere Frequenzlinien und/oder Frequenzbänder in ihrer Frequenz verstellbar sind.

3. Vorrichtung nach einem der vorhergehenden Ansprüche, dadurch gekennzeichnet, daß das Frequenzspektrum innerhalb eines Frequenzintervalls von 1 kHz bis 10 MHz liegt.

4. Vorrichtung nach einem der vorhergehenden Ansprüche, dadurch gekennzeichnet, daß Schall- und/oder Ultraschallwellen von einer oder mehreren Frequenzlinien und/oder Frequenzbänder in ihrer Amplitude (A1 bis A4) und Breite (H1 bis H4) variierbar sind.

5. Vorrichtung nach einem der vorhergehenden Ansprüche, dadurch gekennzeichnet, daß die von der Schallquelle des Behandlungs-Geräts abgestrahlte Leistung bis zu 300 Watt pro cm² beträgt.

6. Vorrichtung nach einem der vorhergehenden Ansprüche, dadurch gekennzeichnet, daß die US-Bestrahlung in Form von Impulsfolgen realisierbar ist.

7. Vorrichtung nach Anspruch 6, dadurch gekennzeichnet, daß die Impulsfolgen in ihrer Impulsbreite (I) und/oder in ihrer Pausendauer (P) verstellbar sind.

8. Vorrichtung nach einem der vorhergehenden Ansprüche 6 oder 7, dadurch gekennzeichnet, daß die Impulsbreite (I) und/oder die Pausendauer (P) der Impulsfolgen zwischen 0,1 s und 120 s variierbar sind.

9. Vorrichtung nach einem der vorhergehenden Ansprüche, dadurch gekennzeichnet, daß ein Schallkopf als Schallquelle insbesondere für eine Unterwasseranwendung wasserdicht ausgebildet ist.

10. Vorrichtung nach einem der vorhergehenden Ansprüche, dadurch gekennzeichnet, daß ein Schallkopf in seiner äußeren Form an die Form von Körperöffnungen und/oder Körperkanäle, wie Nase, Luftröhre, Blutgefäße etc., angepaßt ist.

11. Vorrichtung nach einem der vorhergehenden Ansprüche, dadurch gekennzeichnet, daß die Schallquelle eine Abstrahlfläche aufweist die so groß gewählt ist, so daß ein menschlicher Körper oder zumindest Körperteile wie Kopf, Bauch, etc. als Ganzes bestrahlbar sind.

12. Vorrichtung nach einem der vorhergehenden Ansprüche, dadurch gekennzeichnet, daß ein steuerbarer Führungsmechanismus vorhanden ist, um in einer Relativbewegung zwischen einem Schallkopf und dem zu bestrahlenden Körper bzw. des zu bestrahlenden Körperteils diesen bzw. dieses abzurastern.

13. Vorrichtung nach einem der vorhergehenden Ansprüche, dadurch gekennzeichnet, daß dem Beschallungsgerät (2, 3) ein Teil- oder Vollunterkühlungsaggregat mit Behandlungstisch (1) zugeordnet ist, wobei die Intensität der Unterkühlung einstellbar ist.

14. Vorrichtung nach Anspruch 13, dadurch gekennzeichnet, daß der Unterkühlungsvorrichtung ein Mittel zur selektiven Erwärmung des malignen Gewebes nachgeschaltet ist.

15. Vorrichtung nach einem der vorhergehenden Ansprüche, dadurch gekennzeichnet, daß dem Beschallungsgerät (2, 3) ein Echoanalysator (4) für eine Echoauswertung des beschallten Gewebes zugeordnet ist.

16. Vorrichtung nach einem der vorhergehenden Ansprüche, dadurch gekennzeichnet, daß ein weiteres Beschallungsgerät (8, 9) vorgesehen ist.

17. Vorrichtung nach einem der vorhergehenden Ansprüche, dadurch gekennzeichnet, daß ein Laser-Interferometer (10) vorgesehen ist.

18. Vorrichtung nach Anspruch 15, dadurch gekennzeichnet, daß der Echoanalysator (4) für die Frequenzbestimmung der reflektierten Schallwellen und die Festlegung der Bestrahlungsfrequenzen vorgesehen ist.

19. Vorrichtung nach einem der vorhergehenden Ansprüche, dadurch gekennzeichnet, daß Mittel (7) für die simultane Bestimmung der letalen Bestrahlungsintensität während der Behandlung des malignen Gewebes vorgesehen sind.

## Claims

1. Device for treating diseased cells and malignant tissue in a living body by means of ultrasonic waves with a control and/or regulating unit and an ultrasonic device (2, 3) connected to the control and/or regulating unit with at least one sound source (3) for the selective destruction of cells with a diseased appearance, for example tumour cells, for the production of ultrasonic waves, characterised in that the device for producing sound and/or ultrasound rays has a resonance frequency spectrum typical of the respective cell types which destroys said cells using a selective radiation that is lethal to malignant cells, whereby means for the superpostioning of different frequency lines (F1 to F4) and/or frequency bands are provided.

2. Device according to Claim 1, characterised in that the frequency of one or more frequency lines and/or frequency bands can be varied.

3. Device according to one of the preceding claims, characterised in that the frequency spectrum lies within a frequency interval of 1 kHz to 10 MHz.

4. Device according to one of the preceding Claims, characterised in that sound and/or ultrasound waves of one or more frequency lines and/or frequency bands can be varied in amplitude (A1 to A4) and width (H1 to H4).

5. Device according to one of the preceding Claims, characterised in that the power radiated from the sound source of the treatment device is up to 300 Watt per cm².

6. Device according to one of the preceding Claims, characterised in that the ultrasonic radiation can be in the form of pulse sequences.

7. Device according to Claim 6, characterised in that the pulse sequences can be varied in pulse width (I) and/or pause duration (P).

8. Device according to one of the preceding Claims 6 or 7, characterised in that the pulse width (I) and/or the pause duration (P) of the pulse sequences can be varied between 0.1 s and 120 s.

9. Device according to one of the preceding Claims, characterised in that as a sound source a sound head is designed to be watertight, in particular for underwater use.

10. Device according to one of the preceding Claims, characterised in that a sound head in its outer form is adapted to the shape of body openings and/or body channels, such as the nose, airways, blood vessels etc.

11. Device according to one of the preceding Claims, characterised in that the sound source has a radiation surface that is sufficiently large for a human body or at least body parts such as the head, abdomen etc. to be radiated as a whole.

12. Device according to one of the preceding Claims, characterised in that a controllable guiding mechanism is provided in order to scan the body or body part in a relative movement between a sound head and the body or body part to be exposed to radiation.

13. Device according to one of the preceding Claims, characterised in that a partial or total supercooling unit with a treatment table (1) is assigned to the ultrasonic device (2, 3), whereby the intensity of the supercooling can be varied.

14. Device according to Claim 13, characterised in that the supercooling device is connected after a means for the selective warming of the malignant tissue.

15. Device according to one of the preceding Claims, characterised in that an echo analyser (4) for evaluating the echo of the radiated tissue is assigned to the ultrasonic device (2, 3).

16. Device according to one of the preceding Claims, characterised in that an additional ultrasonic device (8, 9) is provided.

17. Device according to one of the preceding Claims, characterised in that a laser interferometer (10) is provided.

18. Device according to Claim 15, characterised in that the echo analyser (4) for determining the frequency of the reflected sound waves and defining the radiation frequencies is provided.

19. Device according to one of the preceding Claims, characterised in that means (7) for the simultaneous determination of the lethal radiation intensity during the treatment of the malignant tissue are provided.

## Revendications

1. Dispositif pour traiter des cellules morbides et des tissus malins dans le corps vivant à l'aide d'ondes ultrasonores, comportant une unité de commande et/ou de régulation et un appareil d'irradiation par ultrasons (2, 3), qui est relié à l'unité de commande et/ou de régulation et comporte au moins une source acoustique (3) pour détruire de façon sélective des cellules ayant une apparence morbide, par exemple des cellules de tumeurs, pour la production d'ondes ultrasonores, caractérisé en ce que le dispositif servant à produire un rayonnement acoustique et/ou ultrasonore est conçu avec un spectre de fréquences de résonance, qui est typique des types respectifs de cellules et détruit ces cellules, avec utilisation d'une puissance sélective d'irradiation par ultrasons, qui tue des cellules malignes, des moyens étant prévus pour la superposition de raies de fréquences différentes (F1 à F4) et/ou de bandes de fréquences différentes.

2. Dispositif selon la revendication 1, caractérisé en ce que la fréquence d'une ou de plusieurs raies de fréquences et/ou bandes de fréquences peut être décalée.

3. Dispositif selon l'une des revendications précédentes, caractérisé en ce que le spectre des fréquences est situé dans un intervalle de fréquences de 1 kHz à 10 MHz.

4. Dispositif selon l'une des revendications précédentes, caractérisé en ce que les amplitudes (A1 à A4) et les largeurs (H1 à H4) des ondes acoustiques et/ou des ondes ultrasonores d'une ou de plusieurs raies de fréquences et/ou bandes de fréquences peuvent être modifiées.

5. Dispositif selon l'une des revendications précédentes, caractérisé en ce que la puissance émise par la source acoustique de l'appareil de traitement vaut jusqu'à 300 watts par cm².

6. Dispositif selon l'une des revendications précédentes, caractérisé en ce que l'irradiation d'ondes ultrasonores peut être réalisée sous la forme de suites d'impulsions.

7. Dispositif selon la revendication 6, caractérisé en ce que la largeur (I) des impulsions et/ou la durée (P) de pause entre les impulsions des suites d'impulsions sont réglables.

8. Dispositif selon l'une des revendications précédentes 6 ou 7, caractérisé en ce que la largeur (I) des impulsions et/ou la durée de pause (P) entre les impulsions des suites d'impulsions peuvent être modifiées entre 0,1 s et 120 s.

9. Dispositif selon l'une des revendications précédentes, caractérisé en ce qu'une tête acoustique en tant que source acoustique est agencée de manière à être étanche à l'eau notamment dans le cas d'une utilisation avec immersion dans l'eau.

10. Dispositif selon l'une des revendications précédentes, caractérisé en ce que la forme extérieure d'une tête acoustique est adaptée à la forme d'ouvertures corporelles et/ou de conduits corporels tels que le nez, les voies respiratoires, les vaisseaux sanguins, etc.

11. Dispositif selon l'une des revendications précédentes, caractérisé en ce que la source acoustique possède une surface d'irradiation par ultrasons qui est choisie suffisamment grande pour qu'un corps humain ou au moins des parties du corps telles que la tête, le ventre, etc. puissent être irradiées dans leur ensemble.

12. Dispositif selon l'une des revendications précédentes, caractérisé en ce qu'il est prévu un mécanisme de guidage commandable pour, lors d'un déplacement relatif entre une tête acoustique et le corps irradié ou la partie du corps irradiée, explorer selon un balayage de trame ce corps ou cette partie du corps.

13. Dispositif selon l'une des revendications précédentes, caractérisé en ce qu'à l'appareil d'irradiation par ultrasons (2, 3) est associée une unité de surrefroidissement partiel ou complet comportant une table de traitement (1), l'intensité du surrefroidissement étant réglable.

14. Dispositif selon la revendication 13, caractérisé en ce qu'un moyen permettant de chauffer sélectivement le tissu malin est installé en aval du dispositif de surrefroidissement.

15. Dispositif selon l'une des revendications précédentes, caractérisé en ce qu'un analyseur d'échos (4) pour une exploitation d'échos du tissu irradié est associé à l'appareil d'irradiation par ultrasons (2, 3).

16. Dispositif selon l'une des revendications précédentes, caractérisé en ce qu'il est prévu un autre appareil d'irradiation par ultrasons (8, 9).

17. Dispositif selon l'une des revendications précédentes, caractérisé en ce qu'il est prévu un interféromètre à laser (10).

18. Dispositif selon la revendication 15, caractérisé en ce que l'analyseur d'échos (4) est destiné à déterminer la fréquence des ondes sonores réfléchies et à déterminer les fréquences d'irradiation.

19. Dispositif selon l'un des revendications précédentes, caractérisé en ce que des moyens (7) sont prévus pour déterminer simultanément l'intensité létale d'irradiation pendant le traitement du tissu malin.
